# EUROPEAN PATENT APPLICATION

(11) **EP 2 777 564 A1**
(43) Date of publication of application: **17.09.2014**
(21) Application number: 14160278.9
(22) Date of filing: 17.03.2014
(51) Int. Cl.: A61B 17/34, A61B 19/00

(54) **Method and system for an improved quality control of biopsies**

(30) Priority: 15.03.2013 EP 13159592
(71) Applicant: Advanced Medical Diagnostics Holding NV, 1410 Waterloo (BE)
(72) Inventor: Alberts, Arnaud, 1070 Anderlecht Brussels (BE); Hupin, Antoine, 1140 Brussels (BE); Vandersmissen, Eva, 2800 Mechelen (BE); Makowka, Rafal, 1050 Brussels (BE); Huber, Wolfgang, 1050 Ixelles Brussels (BE); Meier, Sven, 1970 Wezembeek-Oppem (BE)
(74) Representative: Brantsandpatents bvba

(57) **Abstract**

The current invention concerns a method for assessing the quality of a biopsy of a tissue, comprising the steps of calculating image frames from ultrasound data obtained during a biopsy procedure on said tissue; identifying within said image frames a set of needle-showing image frames; recording at least part of said set of needle-showing image frames; computing an actual biopsy location and/or an actual biopsy needle trajectory, on the basis of said set of needle-showing image frames and/or on the basis of said ultrasound data; comparing said actual biopsy location with a targeted biopsy location and/or comparing said actual biopsy needle trajectory with a targeted biopsy needle trajectory. The current invention also concerns a system implementing such a method. The systems and methods of the present invention further help to increase the speed of a biopsy procedure.

## Description

### TECHNICAL FIELD

The invention pertains to the technical field of systems and methods for visualizing ultrasound data. The invention can be used to improve or assess the quality of a biopsy.

### BACKGROUND

Performing a biopsy of tissue, e.g. prostate tissue for the detection of carcinogenic cells, can be difficult as a practitioner does not always know where the biopsy needle is and from which region the biopsy sample is taken.

One option to overcome this is by taking ultrasound data during the biopsy, which can be processed and visualized in order to give the practitioner an idea of how the ultrasound probe and the biopsy needle are located with respect to the tissue to be biopsied.

However, such visualization may not always be sufficient, as the biopsy needle may not always be seen by the practitioner as he or she could be focused on performing the procedure rather than on looking at the screen. The practitioner therefore may not be capable of assessing the quality of the biopsy as he may not have seen where the needle has taken a sample.

Furthermore, present day techniques allow for a targeted biopsy, wherein biopsy locations which have an increased probability of containing carcinogenic cells can be identified on the basis of an algorithm for calculating said probability e.g. on the basis of ultrasound data of the tissue to be biopsied.

There remains a need in the art for an improved method and system for assessing and improving the quality of a biopsy. The invention thereto aims to provide such a method and system which aims to resolve at least some of the problems mentioned above.

### SUMMARY OF THE INVENTION

The present invention provides a method for assessing the quality of a biopsy of a tissue, comprising the steps of:
- calculating image frames from ultrasound data obtained during a biopsy procedure on said tissue;
- identifying within said image frames a set of needle-showing image frames;
- recording at least part and preferably all of said set of needle-showing image frames;
- computing an actual biopsy location and/or an actual biopsy needle trajectory, preferably on the basis of said set of needle-showing image frames and/or on the basis of said ultrasound data;
- comparing said actual biopsy location with a targeted biopsy location and/or comparing said actual biopsy needle trajectory with a targeted biopsy needle trajectory.
Furthermore, the present invention provides a system for assessing the quality of a biopsy of a tissue. The system thereto comprises a processing unit arranged to perform the steps of:
- calculating image frames from ultrasound data obtained during a biopsy procedure on said tissue;
- identifying within said image frames a set of needle-showing image frames;
- recording at least part and preferably all of said set of needle-showing image frames;
- preferably obtaining or computing an actual biopsy location or an actual biopsy needle trajectory or both, preferably on the basis of said set of needle-showing image frames and/or on the basis of said ultrasound data;
- preferably comparing said actual biopsy location with a targeted biopsy location and/or comparing said actual biopsy needle trajectory with a targeted biopsy needle trajectory.

A method and system as described here above, is particularly suited to allow a practitioner to assess the quality of a biopsy. The targeted biopsy locations or the targeted biopsy needle trajectories can be computed in advance on the basis of a previous scan, preferably a previous 3D scan, and preferably on the basis of ultrasound data obtained of the tissue which is to be biopsied, preferably in a non-invasive manner. The targeted locations could be computed to be the most relevant locations for e.g. finding carcinogenic cells, and the targeted trajectories could be computed to be e.g. the least invasive for taking a biopsy sample or the most certain to obtain a biopsy sample from a region in the tissue which is computed to contain e.g. carcinogenic cells. Alternatively, the targeted location could be computed in a random or pseudo-random manner, or a specific number of targeted locations could be chosen spread over the tissue using a template or pattern, e.g. 2 biopsy locations in the left base, 2 biopsy locations in the left middle region, 2 biopsy locations in the left apex, etc. of the tissue or organ. It is seen that even the latter scheme can be hard to accomplish by a practitioner, whereby all biopsy samples are taken in a specific region such as the base or the left side of e.g. the prostate, thereby leaving large parts of the prostate unsampled.

By comparing the targeted locations and trajectories with the actual locations and trajectories, the practitioner obtains information about the precision with which the biopsy procedure was performed, and furthermore obtains information about the exact locations which were actually biopsied in view of e.g. a follow-up biopsy procedure. It is clearly of benefit to know whether the actual biopsy locations were near the targeted biopsy locations where e.g. the chances of finding carcinogenic cells are high.

In a further aspect, the present invention provides a method for improving the quality of a biopsy of a tissue, comprising the steps of:
- calculating image frames from ultrasound data obtained during a biopsy procedure on said tissue;
- visualizing said image frames simultaneously with a targeted biopsy location and/or a targeted biopsy needle trajectory;
- identifying within said set of images a subset of needle-showing image frames;
- after a subset of needle-showing image frames has been identified, visualizing said set of image frames simultaneously with a next targeted biopsy location and/or a next targeted biopsy needle trajectory.
Further, the present invention provides a system for improving the quality of a biopsy of a tissue. The system thereto comprises a processing unit arranged to perform the steps of:
- calculating image frames from ultrasound data obtained during a biopsy procedure on said tissue;
- visualizing said image frames simultaneously with a targeted biopsy location and/or a targeted biopsy needle trajectory;
- identifying within said set of images a subset of needle-showing image frames;
- after a subset of needle-showing image frames has been identified, visualizing said set of image frames simultaneously with a next targeted biopsy location and/or a next targeted biopsy needle trajectory.

An important advantage of the above described system and method is that it provides an automated system and an automated method for showing the next target location or trajectory without any interaction necessary from the part of the practitioner. Such a method and system also allows faster procedures because of the automatical switching to a next target.

The two methods and the two systems described here above may be combined. This results in a method whereby e.g. the next targeted location and/or trajectory which is visualized can be computed on the basis of the comparison between the previous actual and targeted biopsy location and/or between the previous actual and targeted biopsy needle trajectory. Thereto, the present invention provides a method for improving the quality of a biopsy of a tissue, comprising the steps of:
- calculating image frames from ultrasound data obtained during a biopsy procedure on said tissue;
- visualizing said image frames simultaneously with a targeted biopsy location and/or a targeted biopsy needle trajectory;
- identifying within said set of images a subset of needle-showing image frames;
- recording at least part of said set of needle-showing image frames;
- computing an actual biopsy location and/or an actual biopsy needle trajectory, preferably on the basis of said set of needle-showing image frames and/or on the basis of said ultrasound data;
- comparing said actual biopsy location with a targeted biopsy location and/or comparing said actual biopsy needle trajectory with a targeted biopsy needle trajectory;
- after a subset of needle-showing image frames has been identified, visualizing said set of image frames simultaneously with a next targeted biopsy location and/or a next targeted biopsy needle trajectory, whereby preferably said next targeted biopsy location and/or said next targeted biopsy needle trajectory are computed taking into account said comparison between said actual biopsy location and said targeted biopsy location and/or taking into account said comparison between said actual biopsy needle trajectory and said targeted biopsy needle trajectory.

### DESCRIPTION OF FIGURES

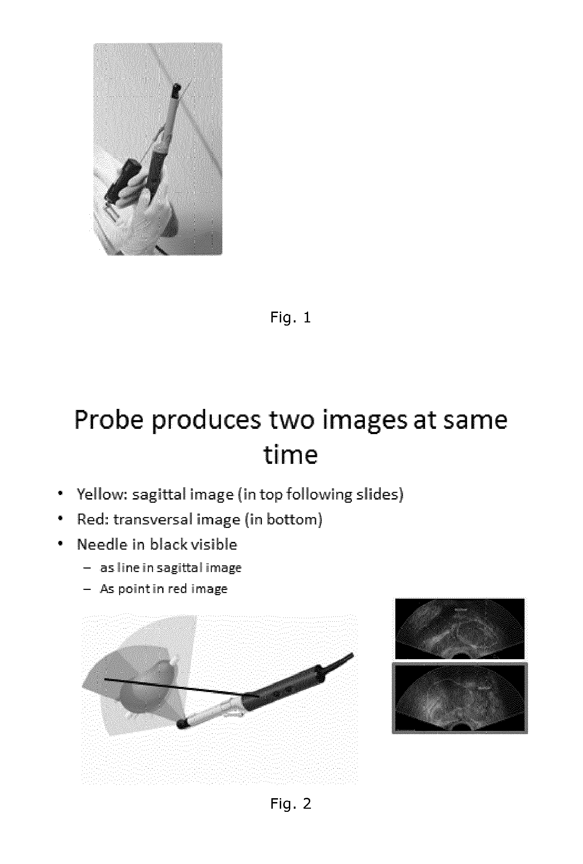

**Figures 1 to 10** illustrate an embodiment of the present invention in which the workings of a method and a system for assessing or improving the quality of a biopsy of a tissue corresponding to a prostate are exemplified on a step by step basis.

### DETAILED DESCRIPTION OF THE INVENTION

The present invention concerns methods and systems for assessing and/or improving the quality of a biopsy of a tissue.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

In a preferred embodiment of the invention, the actual biopsy location and/or the actual biopsy needle trajectory is computed on the basis of said set of needle-showing image frames and/or on the basis of said ultrasound data, in particular on the basis of a subset of said ultrasound data related to said set of needle-showing image frames. Hereby, the algorithm to detect the presence of a needle may be based directly on data obtained from an ultrasound scan or may be based on the images reconstructed on the basis of such an ultrasound scan.

In a preferred embodiment, said identification within said set of images of a subset of needle-showing image frames comprises analyzing said ultrasound data for the presence of a needle, preferably taking into account said targeted biopsy location and/or said targeted biopsy needle trajectory. In particular, the identification may comprise performing an needle detecting algorithm taking into account an expected position of said needle, the expected position preferably being computed on the basis of the targeted biopsy location and/or the targeted biopsy needle trajectory. As the methods and systems of this invention visualize the targeted biopsy location and/or the targeted biopsy needle trajectory, the algorithm for detecting the presence of a needle can be optimized by taking into account the targeted position and/or trajectory.

In a preferred embodiment, the ultrasound data of the tissue is obtained during a biopsy, preferably in a non-invasive manner.

In a preferred embodiment, said image frames are chronologically ordered and/or said subset of needle-showing image frames are chronologically ordered.

In a preferred embodiment, said subset of needle-showing image frames comprises subsequent image frames. This allows for a better computation of the actual biopsy location and/or the actual biopsy needle trajectory.

In a preferred embodiment, said actual biopsy location is compared with said targeted biopsy location by:
- visualizing, preferably simultaneously in an overlay image, said actual biopsy location and said targeted biopsy location; and/or
- providing an indication of mismatch between said actual biopsy location and said targeted biopsy location.

Similarly, in a preferred embodiment, said actual biopsy needle trajectory is compared with said targeted biopsy needle trajectory by:
- visualizing, preferably simultaneously in an overlay image, said actual biopsy needle trajectory and said targeted biopsy needle trajectory; and/or
- providing an indication of mismatch between said actual biopsy needle trajectory and said targeted biopsy needle trajectory.

In a preferred embodiment, said image frames are visualized simultaneously with a targeted biopsy location and/or a targeted biopsy needle trajectory. This allows for e.g. a real-time or nearly real-time monitoring of the tissue and the biopsy needle and for a real-time or nearly real-time comparison by visual inspection of the actual biopsy location and/or the actual biopsy needle trajectory with the targeted biopsy location and/or biopsy needle trajectory.

In a particularly preferred embodiment, all of said set of needle-showing image frames are recorded. This allows for a better assessment of the quality of the biopsy, in particular after the biopsy procedure has completed. The recorded needle-showing frames can be re-viewed and processed after the procedure to more precisely compare e.g. the actual biopsy locations with the targeted biopsy locations. It may not always be possible to assess the quality of the biopsy at the moment of or just after taking a biopsy sample. The needle may, for instance, be only visualized on one or two image frames. If these image frames are visualized at the moment of the procedure, the practitioner may not have seen the images clearly or not at all. Post-procedure inspection of these images can thereby help to assess the quality of the biopsy of the tissue.

In a particularly preferred embodiment, a number of image frames is recorded, said number of image frames corresponding to the ultrasound data obtained during a first period immediately prior to the moment corresponding to said set of needle-showing image frames and/or during a second period immediately after the moment corresponding to said set of needle-showing image frames. This first and/or second period preferably corresponds to at least 1, 2, 3, 4, 5 seconds, more preferably to at least 10 seconds. In this way, essentially a movie can be reconstructed of the taking of a biopsy sample, starting from a few seconds, e.g. 10 seconds, before the sample is taken and lasting until a few seconds, e.g. 5 seconds, afterwards.

The invention is further described by the following non-limiting examples which further illustrate the invention, and are not intended to, nor should they be interpreted to, limit the scope of the invention.

### EXAMPLES

Figures 1-10 illustrate an embodiment of the present invention in which the workings of a method and a system for assessing or improving the quality of a biopsy of a tissue corresponding to a prostate are exemplified on a step by step basis.

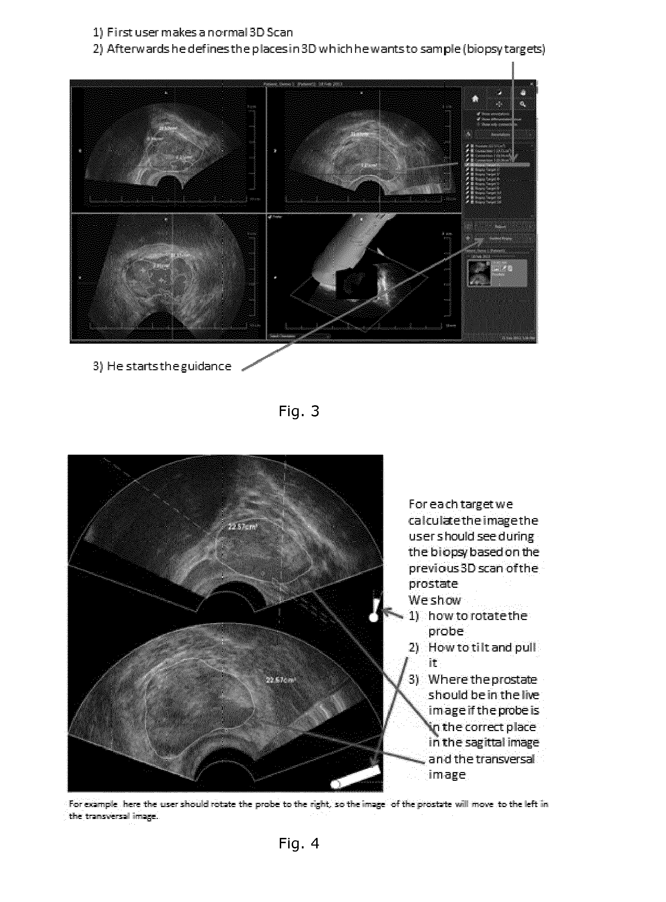

Herein, a probe with a needle (fig. 1) can be used to obtain the data during the biopsy. The probe can be capable of obtaining 3D data from which images can be reconstructed and visualized by showing a sagittal - top images in figs. 1 to 10 - and a transversal image - bottom images in figs. 1-10 - (fig. 2). The needle is expected to show up in these images as a line in the sagittal image and as a point in the transversal image. Targeted biopsy locations and targeted biopsy needle trajectories can be computed on the basis of previous scans of the tissue, e.g. by a previous 3D scan with ultrasound and by an algorithm to detect regions of increased probability of finding carcinogenic cells from ultrasound data (fig. 3). The method of the present invention can then be started, i.e. a first targeted biopsy location can be visualized simultaneously with image frames computed on the basis of ultrasound data obtained during a biopsy of the tissue. This is illustrated in fig. 3, wherein:
1) First, a user makes a normal 3D scan
2) Afterwards, he defines the places in 3D which he wants to sample (biopsy targets)
3) He starts the guidance

Then, as shown in fig. 4, for each target the image the user should see during the biopsy is calculated based on the previous 3D scan of the prostate. The optimal rotation of the probe is visualized by visualizing how to tilt and pull it and where the prostate should be in the live image if the probe is in the correct place in the sagittal image and the transversal image. In the shown example, the user should rotate the probe to the right, so the image of the prostate will move to the left in the transversal image.

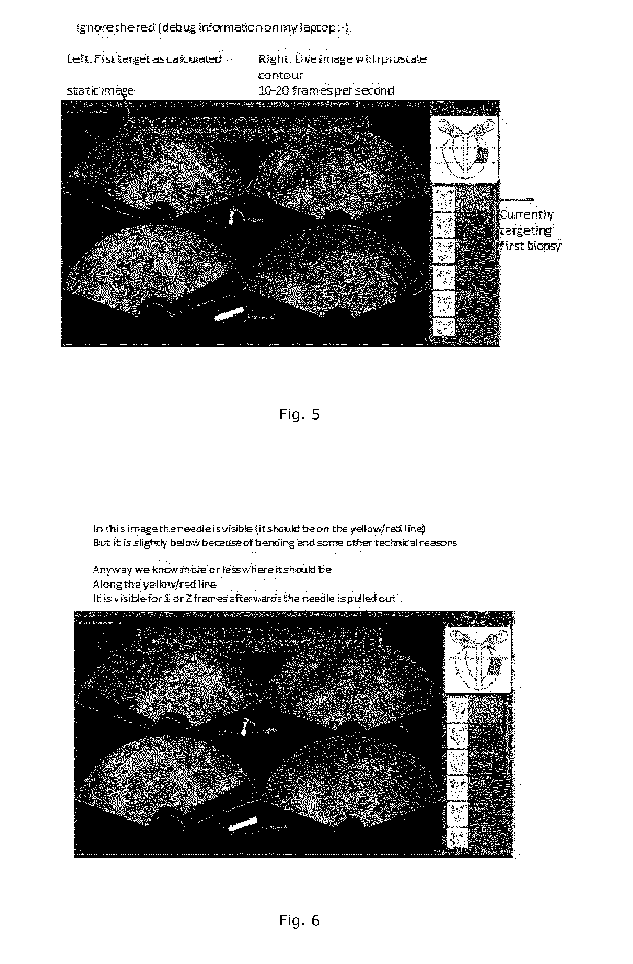

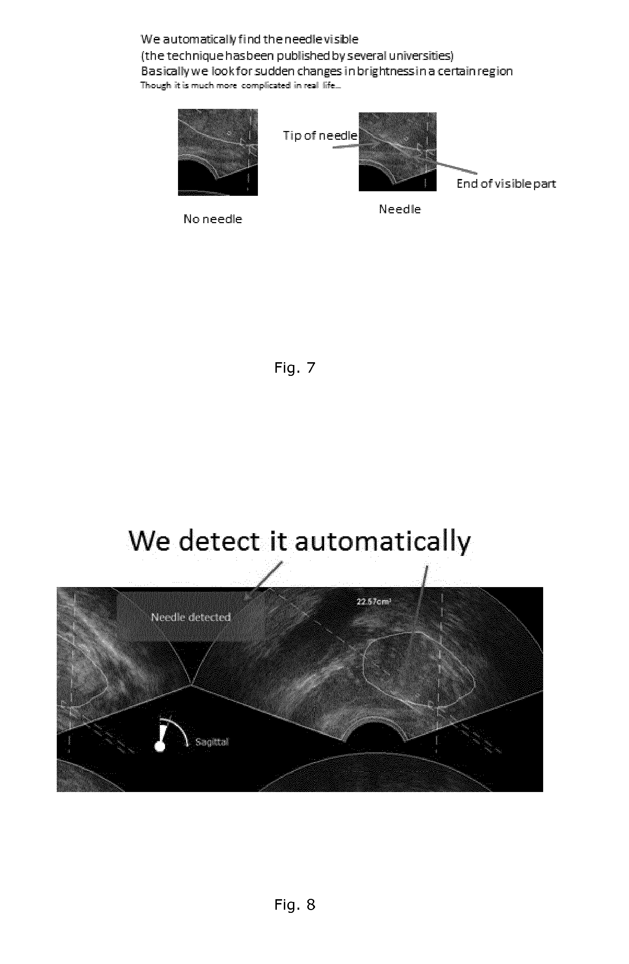

The following steps can be used to give guidance to the biopsy procedure. Hereby (see fig. 5), the first targeted biopsy location is visualized in a static image on the left and the live or recorded image with a prostate contour is visualized at e.g. 10-20 frames per second. Hereby, the first target can be shown as calculated. On the right, a list of targeted biopsy locations can be presented. Hereby, the currently targeted biopsy, i.c. the first target, can be highlighted. In the right image of fig. 6, needle-showing image frames are visualized and the presence of the needle is automatically detected. It is seen on the figure, that the needle is slightly below the targeted trajectory because of bending and some other technical reasons. If the user follows the guidance of the system, one knows more or less the position where the needle should be. In this example, the needle is visible in 1 or 2 subsequent frames before it is removed. The needle is detected automatically (figs. 7-8), i.e. needle-showing image frames are identified automatically. This can be done by an algorithm which basically looks for sudden changes in brightness in a certain region, e.g. in the expected region where the needle may be found. Hereby, frames without needle (fig. 7, left) and frames where at least part of the needle, i.c. from the tip of the needle the the end of the visible part of the needle, can be identified (fig. 7, right). If a frame showing a needle is identified, a message, such as 'Needle detected', can inform the used (fig. 8).

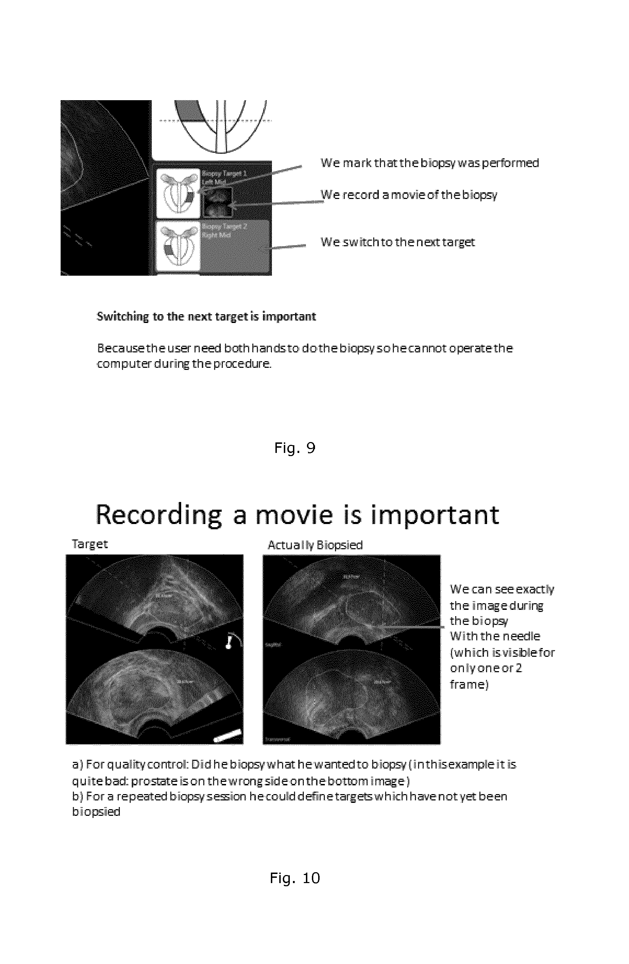

After the needle is detected and the subset of needle-showing image frames are identified, the next targeted biopsy location and targeted biopsy needle trajectory can be visualized automatically (fig. 9). Hereby, we can mark that the biopsy was performed, record a movie of the biopsy, and switch to the next target. Switching to the next target is important because the user need both hands to do the biopsy so he cannot operate the computer during the procedure.

Furthermore, recording a movie, i.e. a set of image frames, preferably the needle-showing image frames, whereby we can see exactly the image during the biopsy with the needle, which may be visible for only on or 2 frames, is important (fig. 10),:
- for quality control: Did the user biopsy what he wanted to biopsy. In this example it is quite bad: prostate is on the wrong side on the bottom image.
- for a repeated biopsy session the user could define targets which have not yet been biopsied.

In the described example (figs. 1-10), the automatic needle detection allows that all steps can be performed automatically without interaction of the user. Furthermore, after the planning step,
- the targets are shown one after the other; and
- the actual biopsies are recorded and a report is created: target vs actual.

## Claims

1. Method for assessing the quality of a biopsy of a tissue, comprising the steps of:
- calculating image frames from ultrasound data obtained during a biopsy procedure on said tissue;
- identifying within said image frames a set of needle-showing image frames;
- recording at least part of said set of needle-showing image frames;
- computing an actual biopsy location and/or an actual biopsy needle trajectory, on the basis of said set of needle-showing image frames and/or on the basis of said ultrasound data;
- comparing said actual biopsy location with a targeted biopsy location and/or comparing said actual biopsy needle trajectory with a targeted biopsy needle trajectory.

2. Method according to claim 1, whereby said actual biopsy location and/or said actual biopsy needle trajectory is computed on the basis of said set of needle-showing image frames and/or on the basis of said ultrasound data.

3. Method according to any of the claims 1 to 2, comprising the steps of:
- obtaining ultrasound data of said tissue during a biopsy in a non-invasive manner.

4. Method according to any of the claims 1 to 3, whereby said identification comprises analyzing said ultrasound data for the presence of a needle, preferably taking into account said targeted biopsy needle trajectory.

5. Method according to any of the claims 1 to 4, whereby said set of image frames is chronologically ordered.

6. Method according to any of the claims 1 to 5, whereby said subset of needle-showing image frames comprises subsequent image frames.

7. Method according to any of the claims 1 to 6, whereby said actual biopsy location is compared with said targeted biopsy location by:
- visualizing, preferably simultaneously in an overlay image, said actual biopsy location and said targeted biopsy location; and/or
- providing an indication of mismatch between said actual biopsy location and said targeted biopsy location.

8. Method according to any of the claims 1 to 7, whereby said actual biopsy needle trajectory is compared with said targeted biopsy needle trajectory by:
- visualizing, preferably simultaneously in an overlay image, said actual biopsy needle trajectory and said targeted biopsy needle trajectory; and/or
- providing an indication of mismatch between said actual biopsy needle trajectory and said targeted biopsy needle trajectory.

9. Method according to any of the claims 1 to 8, comprising the steps of:
- after a subset of needle-showing image frames has been identified, visualizing said set of image frames simultaneously with a next targeted biopsy location and/or a next targeted biopsy needle trajectory.

10. Method according to any of the claims 1 to 9, comprising the steps of:
- visualizing said image frames simultaneously with a targeted biopsy location and/or a targeted biopsy needle trajectory.

11. Method according to any of the claims 1 to 10, whereby all of said set of needle-showing image frames are recorded.

12. System for assessing the quality of a biopsy of a tissue, comprising a processing unit arranged to perform the steps of:
- calculating image frames from ultrasound data obtained during a biopsy procedure on said tissue;
- identifying within said image frames a set of needle-showing image frames;
- recording at least part of said set of needle-showing image frames;
- preferably obtaining or computing an actual biopsy location or an actual biopsy needle trajectory or both, preferably on the basis of said set of needle-showing image frames and/or on the basis of said ultrasound data;
- preferably comparing said actual biopsy location with a targeted biopsy location and/or comparing said actual biopsy needle trajectory with a targeted biopsy needle trajectory.

13. Method for improving the quality of a biopsy of a tissue, comprising the steps of:
- calculating image frames from ultrasound data obtained during a biopsy procedure on said tissue;
- visualizing said image frames simultaneously with a targeted biopsy location and/or a targeted biopsy needle trajectory;
- identifying within said set of images a subset of needle-showing image frames;
- after a subset of needle-showing image frames has been identified, visualizing said set of image frames simultaneously with a next targeted biopsy location and/or a next targeted biopsy needle trajectory.

14. System for improving the quality of a biopsy of a tissue, comprising a processing unit arranged to perform the steps of:
- calculating image frames from ultrasound data obtained during a biopsy procedure on said tissue;
- visualizing said image frames simultaneously with a targeted biopsy location and/or a targeted biopsy needle trajectory;
- identifying within said set of images a subset of needle-showing image frames;
- after a subset of needle-showing image frames has been identified, visualizing said set of image frames simultaneously with a next targeted biopsy location and/or a next targeted biopsy needle trajectory.
